Europäisches Patentamt

(19) European Patent Office

Office européen des brevets

(11) Publication number: **0 221 206**
**B1**

(12) EUROPEAN PATENT SPECIFICATION

(45) Date of publication of the patent specification:
**28.11.90**

(21) Application number: **85306900.3**

(22) Date of filing: **27.09.85**

(51) Int. Cl.⁵: **C07C 11/08**, C07C 2/30, C07C 2/36, B01J 31/14

(54) Process for making butene-1 from ethylene.

(43) Date of publication of application:
**13.05.87 Bulletin 87/20**

(45) Publication of the grant of the patent:
**28.11.90 Bulletin 90/48**

(84) Designated Contracting States:
**BE DE FR GB**

(56) References cited:
**EP-A- 0 114 416**
**EP-A- 0 135 441**
**DE-A- 2 026 246**
**US-A- 3 969 429**

(73) Proprietor: **Indian Petrochemicals Corporation Ltd., P.O. Petrochemicals, Vadodara District Baroda 391346 Gujarat(IN)**

(72) Inventor: **Pillai, Subramania, Dr., Indian Petrochemicals Corporation Limited, P.O. Petrochemicals Vadodara 391 346(IN)**
Inventor: **Sivaram, Swaminathan, Dr., Indian Petrochemicals Corporation Limited, P.O.Petrochemicals Vadodara 391346(IN)**
Inventor: **Ravindranathan, Marayil, Dr., Indian Petrochemicals Corporation Limited, P.O. Petrochemicals Vadodara 391 346(IN)**
Inventor: **Pillai, Muthukumaru, Indian Petrochemicals Corporation Limited, P.O. Petrochemicals Vadodara 391 346(IN)**

(74) Representative: **Collier, Jeremy Austin Grey et al, J.A.Kemp & Co. 14, South Square Gray's Inn, London WC1R 5EU(GB)**

## Description

The present invention relates to a process for the manufacture of butene-1 from ethylene. More particularly, the invention relates to a process for the dimerisation of ethylene to provide butene-1 of high yield and selectivity and with substantially no undesirable by-product formation of butene-2 or of hexenes, octenes and other higher oligomers or of high molecular weight polymers.

It has been hitherto known that the polymerisation of ethylene to butene-1 can be effected by employing as a catalyst for the polymerisation a mixture of a tetra-alkyl titanate and an aluminium alkyl. Unfortunately, the prior art processes suffer from the basic drawback of the formation of undesirable by-products along with the butene-1. These undesirable by-products include the formation in appreciable amounts of butene-2 which is extremely difficult to separate in a convenient manner from the desired butene-1. Other undesirable by-products formed along with butene-1 are hexenes, octenes and other higher oligomers and high molecular weight polymers. The disadvantages of the prior art will be more obvious from the following description which gives details of the known processes and their drawbacks in terms of undesirable by-product formation.

Belgian Patent No.540136 granted to K.Ziegler discloses the use of a catalyst comprising a mixture of tetra-n-butyl titanate and an aluminium alkyl. However, of the products resulting from the reaction, only 90% were butene of which selectivity to butene-1 was as low as 67%.

In general, the prior art reports the formation of by-product butene-2 during the dimerisation of ethylene to butene-1 when the catalytic process is carried out at temperature in excess of 100°C, at high operational pressures and for an extended residence time of more than six hours. This is specifically illustrated by the disclosure in Canadian Patent No.653326 granted to T.E.C. Knee. This patent discloses a process of dimerising ethylene to butene-1 employing as catalyst a mixture of tetra-n-butyl titanate and aluminium tri-isobutyl at a pressure of 33 kg/cm2 and at a temperature of 75°C to 125°C in a tubular reactor with a short residence time. Specifically to avoid the formation of butene-2, Example 2 of this Canadian patent prescribed the reduction of reaction temperature to 75°C but employed a high operating pressure of 33 kg/cm2. Under these altered conditions, the conversion to butenes was approximately 92% with an excellent selectivity thereof to butene-1 of 99%. However, the drawbacks were twofold. In the first place, 8% of the product remained unaccounted for. Secondly, the process produced about 5% ethylene oligomers such as hexenes and octenes which represented undesirable impurities. Another significant factor as expressed in Example 3 of the Canadian patent was that the aluminium-titanate molar ratio for the catalyst was 3.

According to U.S. Patent No.3879485 in the name of G.P.Belov et al, there is described a process for producing butene-1 from ethylene in the presence of a complex catalyst consisting of titanium alcoholates of the formula (RO)₃ Ti R' - Al R"₂ OR and alkyl aluminium of the formula Al R₂"R' where R is an alkyl radical having from 2 to 4 carbon atoms, R'= R or H and R" is the same as R. However, this process gives rise to by-products in the form of higher oligomers and polymers though it is claimed that such by-products do not exceed 5% of the total ethylene converted.

U.S. Patent No.4101600 granted to V.I.Zhukov describes a process for the catalytic dimerisation of ethylene to butene in which the catalyst comprises a mixture of tetra-n-alkoxy titanium and trialkyl aluminium and wherein the catalytic mixture is pretreated with an ethylene-hydrogen mixture. Unfortunately, although this process suppressed polymer formation and improved conversion to butenes, the selectivity of the product to butene-1 was low and what was more, as much as 10% to 30% by weight of hexenes were found to be present.

Czechoslovakian Patent No. 179183 describes the use of pentane and/or isopentane as solvents for a tetra-n-butyl titanate-triethyl aluminium catalyst system employed in a process for the conversion of ethylene to butene-1. The patent reports that the weight ratio of the solvent to the catalyst should be from 10-1000: 1. However, the process suffers the drawback that hexenes and higher boiling olefins are formed along with butene-1 during the dimerisation reaction.

Another process for the conversion of ethylene to butene-1 is described in Japanese Patent No. 82 169 430 where the catalyst is a system of tetra-n-butyl titanate and triethyl aluminium employed at 70°C. According to a preferred feature of this patent, the catalyst system may be pre-treated in hydrogen, oxygen, nitrogen or a suitable inert gas. Where no pre-treatment is effected, the process achieves an 80% conversion of ethylene to butene-1. With the pre-treatment of the catalyst, the conversion goes up to 85%. In either instance, however, the rest of the products are undesirable hexenes and higher oligomers.

European Specification 0114416A describes a process for producing butene-1 by dimerising ethylene by contacting it with a catalyst containing an alkyl titanate and an organic aluminium compound at a temperature of 90 to 200°C.

The product contain small but significant amounts of butene-2.

German Specification 2026246 describes a process for dimerising alpha-olefins of 2 to 4 carbon atoms by contacting them with a catalyst comprising an alkyl aluminium compound, a titanate, and a phosphine. When the olefin is ethylene, the product contain small amounts of polymer.

It has been the applicants' object to overcome the drawbacks and disadvantages of the prior art through the provision of a simplified process for the dimerisation of ethylene to butene-1. Stated more basically, the applicants' aim is a process which provides butene-1 from ethylene in greater yield and selectivity with the absence of undesirable by-products of the reaction. The applicants have proceeded on the basis that the selectivity of butene-1 in the yield from any process for its production is directly related to reaction parameters and to the presence of undesirable impurities in the reaction.

As a result of their efforts and investigations, the applicants have been able to establish that the attainment of such a desired process depends on two essential criteria. The first of these is the discovery of an optimum weight ratio of aluminium to titanium in the catalyst employed for the dimerisation reaction and the relation of such weight ratio to operational pressures. In a departure from prior art processes, which employ high temperatures and pressures for their reactions, the applicants have for reason of the obvious advantages accruing therefrom attempted to dimerise ethylene at considerably lower temperatures and pressures. In attempting this, they have found that it is possible to produce butene-1 with high selectivity and conversion when working at temperatures lower than 35°C and pressures of less than 15 kg/cm² (14.7 bars). What makes this possible and what represents the significant discovery the applicants have made is the selection of an optimum weight ratio for the aluminium-titanium catalyst employed in the dimerisation reaction. This ratio can vary within only a narrow range above or below which there are adverse effects on both the rate of reaction and the selectivity of the desired product.

The second criterion the applicants have established concerns the purity of the catalyst employed. In an earlier work published in "CHEMTECH", 746 (1981), G.Henrici Olive and S. Olive reported that the presence of chloride ligands on titanium or aluminium leads to oligomerisation and polymerisation. Investigating this further, the applicants have found that even innocuous chloride ions or other polar compounds commonly encountered as impurities in titanium alkoxides can lead to oligomer and polymer formation. Thus, the accidental introduction of polar impurities in titanium alkoxides or the incomplete removal of chloride ions normally present in titanium alkoxides gives rise to the formation of undesirable oligomers and polymers as by-products during the employment of an aluminium-titanium catalyst in the dimerisation of ethylene to butene-1. It follows, therefore, that by employing as catalyst an aluminium-titanium system which has been subjected initially to a thorough purification for removal of chloride ions and other polar compounds, the formation of undesirable oligomer and polymer by-products during the dimerisation reaction could be substantially obviated.

The conclusion drawn by the applicants is vindicated by U.S. Patent No.3969429. Example 1 of this U.S. Patent describes the dimerisation of ethylene to butene-1 at a temperature of 40°C and an ethylene pressure of 12 kg/cm² (11.8 bar). The reaction was effected in n-heptane as solvent over a period of about two hours. The ethylene conversion is not mentioned but the process of the patent employs a catalyst having an aluminium-titanium ratio of 8.6 resulting in the formation of approximately 5% of higher olefins and polymers. The catalyst ratio of Example 1 of U.S. Patent No.3969429 falls outside the range of weight ratios which the applicants have found to be optimum for the dimerisation reaction.

Accordingly, the present invention provides a process for the dimerisation of ethylene to butene-1 which comprises contacting ethylene at a temperature from 0 to 80°C under a pressure of from 1 to 15 kg/cm² (0.98 to 14.7 bar) for 1 to 5 hours with a catalyst which is free of chloride ions and other polar compounds and comprises a mixture iof tetra-alkoxy titanium and trialkyl aluminium present in said mixture in a weight ratio of aluminium of from 1 to 7.5, and thereafter separating and recovering the butene-1 so produced.

The tetra-alkoxy titanium component of the catalyst employed in the process of this invention can be represented by the general formula $Ti(OR)_4$ wherein R is an alkyl, isoalkyl, aryl or arylalkyl group having from 1 to 10 carbon atoms. Preferably, R in this formula is butyl or ispropyl.

The trialkyl aluminium component of the catalyst employed can be represented by the general formula $AlR_3$ wherein one or more of R is an alkyl, aryl or alkoxy group having from 1 to 6 carbon atoms. Preferably, R in this formula is ethyl or isobutyl. According to a preferred feature of the invention, contact between the aluminium-titanium catalyst and the ethylene for purposes of reaction is brought about by saturating the catalyst mixture with ethylene.

The reaction is conveniently effected in the presence of a hydrocarbon solvent. These solvents can be selected from paraffinic hydrocarbons having from 5 to 14 carbon atoms, chlorinated hydrocarbons having from 6 to 8 carbon atoms and aromatic hydrocarbons having from 6 to 8 carbon atoms. The hydrocarbon solvents most preferred for the reaction are hexane and heptane.

According to a preferred feature of the invention, the catalyst components of tetra-alkoxy titanium and trialkylaluminium are mixed together at a temperature of about 25°C to 27°C and such catalyst mixture can include in addition a polar modifier. The use of such polar modifiers has been found to suppress the formation of high molecular weight polymers during the dimerisation reaction. However, while the inclusion of the modifiers actively suppresses polymer formation, their presence also reduces the activity of the catalyst. Accordingly, the sequence of addition of the modifier to the catalyst mixture has a profound bearing on the efficacy and efficiency of the catalyst and of the dimerisation reaction. Thus the polar modifier is added to the trialkylaluminium followed by addition of the tetra-alkoxy titanium.

Preferably, the polar modifier is present in said catalyst mixture in a molar ratio of approximately 1.1 with respect to the tetra-alkoxy titanium and in a molar ratio of approximately 0.1 with respect to the trialkyl aluminium.

The polar modifiers employed may be selected from alkyl or aryl phosphites or phosphines having the general formula $PR_3$, wherein R is a phenoxy, alkyl-substituted phenoxy, phenyl or alkyl group. The modifiers can also be selected from nitrogen compounds of the general formula $NR_3$, wherein R is

an ethyl radical, from aromatic nitrogen-containing compounds such as pyridines, 4-alkyl substituted pyridines, 4-amino substituted pyridines and 4-alkylamino substituted pyridines or from water.

The reaction of the process of the present invention is effected at a temperature of from 0°C to 80°C under a pressure of from 1 to 15 kg/cm² (1.0 to 14.7 bar) over a period of from 1 to 5 hours. Particularly preferred reaction temperatures and times are from 25°C to 35°C for a period of about 1 hour.

Within the scope of such overall reaction conditions, it is preferred when the weight ratio of aluminium to titanium is approximately 4.3 for the reaction to be effected at a pressure of less than 5 kg/cm² (4.9 bar) and when said weight ratio is approximately 6.4 for the reaction to be effected at a pressure greater than 5 kg/cm² (4.9 bar).

After the dimerisation reaction is complete or when further reaction is not desired, alcohol can be added to the reaction mixture in order to terminate the reaction. Such alcohol can be represented by the general formula ROH wherein R is an alkyl group containing from 1 to 4 carbon atoms. Preferred alcohols include methanol and isopropanol. Once the reaction is complete, the desire butene-1 can be recovered in any preferred manner, for instance by evaporating the other components of the final reaction mixture.

The invention will now be described more specifically in the following non-limitative examples which illustrate preferred embodiments of the process of this invention. In these examples, the terms "ethyl", "butyl", "isobutyl", "propyl", "isopropyl", "phenyl" and "phenoxy" have, for convenience, been abbreviated and expressed as "Et", "Bu", "iBu", "Pr", "iPr", "Ph" and "OPh", respectively.

EXAMPLE 1

A glass reactor having 250 ml capacity and equipped with a magnetic stirrer and a condenser was charged with 80 ml heptane saturated with ethylene to a pressure of 1 kg/cm² (1.0 bar) at a temperature of 26°C. 0.1003 g. of Ti(n-BuO)$_4$ and 0.2505 g of AlEt$_3$ in a w/w ratio of Al : Ti of 4.29 were mixed under an atmosphere of nitrogen in heptane as diluent and then added to the reactor. The initial dimerisation rate was 3.36 x10$^{-2}$ moles/litre min. After 120 minutes, 3 ml of methanol were added to terminate the reaction. It was observed that 51% of the ethylene had been converted exclusively into butene-1. No hexenes or octenes or higher molecular weight polymers were formed.

EXAMPLE 2

Under the conditions of Example 1, 0.1680 g. of Ti(n-BuO)$_4$ and 0.2505 g. of AlEt$_3$ (Al : Ti = 3.64) were mixed and injected into the reactor. The initial rate of dimerisation was 1.479 x 10$^{-2}$ moles/litre min. On terminating the reaction after 120 minutes, it was observed that 41% of the ethylene had been converted exclusively to butene-1.

EXAMPLE 3

Under the conditions of Example 1, 0.0939 g. of Ti(n-BuO)$_4$ and 0.334 g. of AlEt$_3$ were used (Al : Ti = 5.99). The initial rate of dimerisation was 3.44 x 10$^{-3}$ moles/litre min. After 120 minutes, it was observed that 36% of the ethylene had been converted exclusively to butene-1.

EXAMPLE 4

Under the conditions of Example 1, 0.1474 g. of Ti(n-BuO)$_4$ and 0.2406 g. of AlEt$_3$ (Al : Ti = 4.29) were used. The initial rate of dimerisation was 0.28 x 10$^{-3}$ moles/litre min. The reaction was run for 120 minutes and yielded 16.3% of butene-1.

EXAMPLE 5

Under the conditions of Example 1, dimerisation of ethylene was carried out with 0.1246 g. of Ti(n-BuO)$_4$ and 0.5533 g. of Al(iBu)$_3$ (Al: Ti = 4.29). The initial rate of dimerisation was 4.91 x 10$^{-3}$ moles/litre min. and the butene-1 conversion 31%.

EXAMPLE 6

Under the conditions of Example 1, benzene was used as a diluent with 0.1096 g. of Ti (n-BuO)$_4$ and 0.2755 g. of AlEt$_3$ (Al: Ti = 4.22). The initial rate of dimerisation was 1.694 x 10$^{-3}$ moles/litre min. and the conversion to butene-1 was 28%.

EXAMPLE 7

Under the conditions of Example 1, petroleum ether having a boiling range of from 40°C to 60°C was used as diluent for 0.1176 g. of Ti(n-BuO)$_4$ and 0.3006 g. of AlEt$_3$ (Al : Ti = 4.29). The initial rate of dimerisation was 5.54 x 10$^{-2}$ moles/litre min. and 32% of the ethylene was converted exclusively to butene-1.

EXAMPLE 8

Under the conditions of Example 1, p-xylene was used as diluent for 0.111 g. of Ti(n-BuO)$_4$ and 0.2839 g. of AlEt$_3$ (Al: Ti = 4.29). The initial rate of dimerisation was 3.26 x 10$^{-3}$ moles/litre min. After 120 minutes, 29% of the ethylene had been converted to butene-1.

EXAMPLE 9

Under the conditions of Example 1, dimerisation was carried out with 0.2123 g. of Ti(n-BuO)$_4$ and 0.25428 g. of A1Et$_3$ at 34°C. The initial rate of the reaction was 8.675 x 10$^{-3}$ moles/litre min. After 120 minutes, 29% of the ethylene had been converted to butene-1.

EXAMPLE 10

Under the conditions of Example 1, 0.1221 g. of Ti(n-BuO)$_4$ and 0.2131 g. of AlEt$_3$ (Al: Ti = 4.29) were used for the dimerisation of ethylene at 44°C. After 120 minutes, 25% of the ethylene had been converted to butene-1.

## EXAMPLE 11

Under the conditions of Example 1, 0.0949 g. of $Ti(n-OPr)_4$ and 0.2923 g. of $AlEt_3$ were used at 28°C. (Al : Ti = 4.29). The initial rate of dimerisation was $8.0419 \times 10^{-4}$ moles/litre min. and the conversion of the ethylene to butene-1 after 120 minutes was 16%.

## EXAMPLE 12

Under the conditions of Example 1, 0.0907 g. of $Ti(OPh)_4$ and 0.1837 g. of $AlEt_3$ were used (Al : Ti = 4.29). The initial rate of the reaction was $7.404 \times 10^{-3}$ moles/litre min. After 120 minutes, 43% of the ethylene had been converted to butene-1.

## EXAMPLE 13.

0.6997 g. of $Ti (n-BuO)_4$ and 2.23 g. of $AlEt_3$ (Al : Ti = 5.35) were mixed in a 500 ml addition funnel with the same volume of heptane. The catalyst solution was charged into a steel reactor of 2l capacity and ethylene was fed into the reactor at a temperature of 33°C. to a pressure of 12.5 kg/cm2 (12.2 bar). The reaction was run for 1 hour under agitation and terminated by the addition of 15 ml of n-butanol. The reaction yielded an 81% conversion of the ethylene to butene-1. 1.26% of the ethylene was converted to high molecular weight polymers.

## EXAMPLE 14

Under the conditions of Example 13, 0.6552 g. of $Ti(n-BuO)_4$ and 2.505 g. of $AlEt_3$ (Al : Ti = 6.414) were used. On terminating the reaction after 1 hour, it was found that 91.9% of the ethylene had been converted to butene-1. 2.1% of ethylene was converted to high molecular weight polymers.

## EXAMPLE 15

Under the conditions of Example 13, 0.6599 g. of $Ti(n-BuO)_4$ and 2.9255 g. of $AlEt_3$ (Al: Ti = 7.47) were used. On terminating the reaction after 1 hour, 86% of the ethylene had been converted to butene-1 and 6.6% to polymers.

## EXAMPLE 16

Under the conditions of Example 13, 0.6488 g. of $Ti(n-BuO)_4$ and 2.4215 g. of $AlEt_3$ (Al : Ti = 6.4) were used at 7.5 kg/cm2 (7.4 bar) pressure of ethylene. The conversion of ethylene to butene-1 after 1 hour's reaction was 89%. There was no polymer formation in this experiment.

## EXAMPLE 17

Under the conditions of Example 13, 0.6452 g. of $Ti(n-BuO)_4$ and 2.505 g. of $AlEt_3$ (Al : Ti = 6.4) were charged into the reactor at 9.5 kg/cm2 (9.3 bar) pressure of ethylene. The conversion of the ethylene to butene-1 and polymers were 93.1% and 1.2% respectively after 1 hour's reaction.

## EXAMPLE 18

Under the conditions of Example 13, 0.5328 g. of $Ti(n-BuO)_4$ and 2.3214 g. of $AlEt_3$ (Al : Ti = 7.47) were used at 7.5 kg/cm2 (7.4 bar) pressure of ethylene. After a reaction period of 1 hour, 36% of the ethylene had been converted to butene-1.

## EXAMPLE 19

Under the conditions of Example 13, 0.6535 g. of $Ti(n-BuO)_4$ and 4.335 g. of $Al(i-Bu)_3$ (Al : Ti = 6.414) were used at 7.5 kg/cm2 (7.4 bar) pressure of ethylene. The reaction run for 1 hour gave 98.8% conversion of the ethylene to butene-1 and 1.2% to polymers.

## EXAMPLE 20

Under the conditions of Example 13, 0.6632 g. of $Ti(n-BuO)_4$ and 2.53 g. of $AlEt_3$ (Al: Ti = 6.414) and 0.7586 g. of $P(OPh)_3$ in a molar ratio of $P(OPh)_3$ : $AlEt_3$ of 0.1 are mixed in the above-mentioned order and charged into the reactor at 7.5 kg/cm2 (7.4 bar) pressure of ethylene. At the end of 1 hour's reaction, 40.4% of the ethylene had been converted to butene-1 and 0.2% to polymers.

## EXAMPLE 21

Under the conditions of Example 13, 0.6347 g. of $Ti(n-BuO)_4$, 0.7369 g. of $P(OPh)_3$ and 2.42 g. of $AlEt_3$ (Al : Ti = 6.414 and $P(OPh)_3$ : $AlEt_3$ = 0.1) were mixed in the above-mentioned order and charged into the reactor at 7.5 kg/cm2 (7.4 bar) pressure of ethylene. At the end of a reaction time of 1 hour, 9.3% of the ethylene had been converted to butene-1 and 0.03% to polymers.

## EXAMPLE 22

Under the conditions of Example 13, 0.735 g. of $P(OPh)_3$, 2.55 g. of $AlEt_3$ and 0.6663 g. of $Ti(n-BuO)_4$ ($P(OPh)_3$ : $AlEt_3$ = 0.1 and Al : Ti = 6.414) were mixed in the above-mentioned order and charged into the reactor at 7.5 kg/cm2 (7.4 bar) pressure of ethylene. After 1 hour's reaction, 74% of the ethylene had been converted to butene-1 and 0.9% to polymers.

## EXAMPLE 23

Under the conditions of Example 13, 1.7955 g. of tri-p-nonylphenyl phosphite as polar modifier, 2.54 g. of $AlEt_3$ and 0.6654 g. of $Ti(n-BuO)_4$ (modifier : $AlEt_3$ = 0.12 and Al : Ti = 6.414) were mixed in the above-mentioned order and charged into the reactor. The reaction was run for 1 hour and gave a 28% conversion of the ethylene to butene-1. No high molecular weight polymers were formed.

## EXAMPLE 24

Under the conditions of Example 13, 0.4954 g. of $PBu_3$, 2.5 g of $A1Et_3$ and 0.6440 g. of $Ti-(n-BuO)_4$ ($PBu_3 : AlEt_3 = 0.11$ and $Al : Ti = 6.414$) were mixed in the above-mentioned order and charged into the reactor. After reaction for 1 hour, 70% of the ethylene had converted into butene-1 with complete suppression of polymer formation.

## EXAMPLE 25

Under the conditions of Example 13, 0.2875 g. of $NEt_3$, 2.5 g. of $AlEt_3$ and 0.6653 g. of $Ti(n-BuO)_4$ ($NEt_3 : AlEt_3 = 0.13$ and $Al : Ti = 6.414$) were mixed in the above-mentioned order and charged into the reactor. After 1 hour's reaction, 31% of the ethylene had been converted to butene-1 and 0.04% to polymer.

The process of the present invention evinces a number of advantages over the prior art. These include a conversion of ethylene to butene-1 in a yield of greater than 95% with a selectivity to butene-1 of more than 99%. The conditions of reaction are mild compared to hitherto known processes and substantially no higher polymers and oligomers of ethylene such as hexenes and octenes are formed.

## Claims

1. A process for the dimerisation of ethylene to butene-1 which comprises contacting ethylene at a temperature from 0° to 80°C under a pressure of from 1 to 15 $kg/cm^2$ (0.98 to 14.7 bar) for 1 to 5 hours with a catalyst which is free of chloride ions and other polar compounds and comprises a mixture of tetra-alkoxy titanium and trialkyl aluminium present in said mixture in a weight ratio of aluminium to titanium of from 1 to 7.5 and thereafter separating and recovering the butene-1 so produced.

2. A process as claimed in claim 1, wherein said tetra-alkoxy titanium has the general formula $Ti(OR)_4$ wherein R is an alkyl, isoalkyl, aryl or arylalkyl group having from 1 to 10 carbon atoms.

3. A process as claimed in claim 2 wherein R in the formula $Ti(OR)_4$ is butyl or isopropyl.

4. A process as claimed in any of claims 1 to 3, wherein said trialkyl aluminium has the general formula $Al(R)_3$ wherein one or more of R is an alkyl, aryl or alkoxy group having from 1 to 6 carbon atoms.

5. A process as claimed in claim 4, wherein R in the formula $Al(R)_3$ is ethyl or isobutyl.

6. A process as claimed in any of claims 1 to 5, wherein contact between said catalyst and said ethylene is effected by saturating said catalyst mixture with said ethylene.

7. A process as claimed in any of claims 1 to 6, wherein the reaction is effected in the presence of a hydrocarbon solvent.

8. A process as claimed in claim 7, wherein said solvent is selected from paraffinic hydrocarbons having from 5 to 14 carbon atoms, chlorinated hydrocarbons having from 6 to 8 carbon atoms, and aromatic hydrocarbons having 1 to 8 carbon atoms.

9. A process as claimed in claim 7 or 8, wherein said hydrocarbon solvent is hexane or heptane.

10. A process as claimed in any of claims 1 to 9, wherein said tetra-alkoxy titanium is mixed with said trialkyl aluminium at a temperature of about 25°C to 27°C.

11. A process as claimed in any of claims 1 to 10, wherein the said reaction is effected at a temperature of from 25°C to 35°C over a period of about 1 hour.

12. A process as claimed in any of claims 1 to 11, wherein when the weight ratio of aluminium to titanium is approximately 4.3, said reaction is effected at a pressure of less than 5 $kg/cm^2$ (4.9 bar).

13. A process as claimed in any of claims 1 to 11, wherein when said weight ratio of aluminium to titanium is approximately 6.4, said reaction is effected at a pressure greater than 5 $kg/cm^2$ (4.9 bar).

14. A process as claimed in any of claims 1 to 13, wherein said mixture of tetra-alkoxy titanium and trialkyl aluminium includes in addition a polar modifier, and said polar modifier is added to said trialkyl aluminium followed by addition of said tetra-alkoxy titanium to form said mixture.

15. A process as claimed in claim 14, wherein said polar modifier is present in said catalyst mixture in a molar ratio of approximately 1.3 with respect to said tetra-alkoxy titanium.

16. A process as claimed in claim 14 or 15, wherein said polar modifier is present in said catalyst mixture in molar ratio of approximately 0.1 with respect to said trialkyl aluminium.

17. A process as claimed in any of claims 14 to 16, wherein said polar modifier is selected from alkyl or aryl phosphites or phosphines having the general formula $PR_3$, wherein R is phenoxy, alkyl-substituted phenoxy, phenyl or alkyl group.

18. A process as claimed in any of claims 14 to 16, wherein said polar modifier is selected from nitrogen compounds of the general formula $NR_3$, wherein R is an ethyl radical, from aromatic nitrogen containing compounds such as pyridines, 4-alkyl substituted pyridines, 4-amino substituted pyridines and 4-alkylamino substituted pyridines or from water.

19. A process as claimed in any of claims 1 to 18, wherein alcohol is added to said reaction mixture in order to terminate the reaction.

20. A process as claimed in claim 19, wherein said alcohol has the general formula ROH, wherein R is an alkyl group containing from 1 to 4 carbon atoms.

## Patentansprüche

1. Verfahren zur Dimerisation von Ethylen zu Buten-1, dadurch gekennzeichnet, daß man Ethylen bei einer Temperatur von 0° bis 80°C und einem Druck von 1 bis 15 $kg/cm^2$ (0.98 bis 14.7 bar) während 1 bis 5 Stunden mit einem Katalysator in Kontakt bringt, der frei ist von Chloridionen und anderen polaren Verbindungen und eine Mischung aus Tetraalkoxytitan und Trialkylaluminium umfaßt, die in dieser Mischung in einem Gewichtsverhältnis von Aluminium zu Titan von 1 bis 7.5 vorhanden sind, und danach das so hergestellte Buten-1 abtrennt und gewinnt.

2. Verfahren nach Anspruch 1, dadurch gekennzeichnet, daß das Tetraalkoxytitan die allgemeine Formel Ti(OR)$_4$ besitzt, worin R eine Alkyl-, Isoalkyl-, Aryl- oder Aralkylgruppe mit 1 bis 10 Kohlenstoffatomen ist.

3. Verfahren nach Anspruch 2, dadurch gekennzeichnet, daß R in der Formel Ti(OR)$_4$ Butyl oder Isopropyl ist.

4. Verfahren nach einem der Ansprüche 1 bis 3, dadurch gekennzeichnet, daß das Trialkylaluminium die allgemeine Formel Al(R)$_3$ besitzt, worin einer oder mehrere der Reste R eine Alkyl-, Aryl oder Alkoxygruppe mit 1 bis 6 Kohlenstoffatomen ist.

5. Verfahren nach Anspruch 4, dadurch gekennzeichnet, daß R in der Formel Al(R)$_3$ Ethyl oder Isobutyl ist.

6. Verfahren nach einem der Ansprüche 1 bis 5, dadurch gekennzeichnet, daß der Kontakt zwischen dem Katalysator und dem Ethylen durch Sättigung der Katalysatormischung mit dem Ethylen herbeigeführt wird.

7. Verfahren nach einem der Ansprüche 1 bis 6, dadurch gekennzeichnet, daß die Umsetzung in Gegenwart eines Kohlenwasserstoff-Lösungsmittels durchgeführt wird.

8. Verfahren nach Anspruch 7, dadurch gekennzeichnet, daß das Lösungsmittel ausgewählt ist aus Paraffinkohlenwasserstoffen mit 5 bis 14 Kohlenstoffatomen, chlorierten Kohlenwasserstoffen mit 6 bis 8 Kohlenstoffatomen, und aromatischen Kohlenwasserstoffen mit 1 bis 8 Kohlenstoffatomen.

9. Verfahren nach Anspruch 7 oder 8, dadurch gekennzeichnet, daß das Kohlenwasserstoff-Lösungsmittel Hexan oder Heptan ist.

10. Verfahren nach einem der Ansprüche 1 bis 9, dadurch gekennzeichnet, daß das Tetraalkoxytitan mit dem Trialkylaluminium bei einer Temperatur von ca. 25°C bis 27°C gemischt wird.

11. Verfahren nach einem der Ansprüche 1 bis 10, dadurch gekennzeichnet, daß die Umsetzung bei einer Temperatur von 25°C bis 35°C während einer Dauer von ca. 1 Stunde durchgeführt wird.

12. Verfahren nach einem der Ansprüche 1 bis 11, dadurch gekennzeichnet, daß das Gewichtsverhältnis von Aluminium zu Titan ca. 4.3 ist, und die Reaktion bei einem Druck von weniger als 5 kg/cm$^2$ (4.9 bar) durchgeführt wird.

13. Verfahren nach einem der Ansprüche 1 bis 11, dadurch gekennzeichnet, daß das Gewichtsverhältnis von Aluminium zu Titan ca. 6.4 ist, und die Reaktion bei einem Druck von größer als 5 kg/cm$^2$ (4.9 bar) durchgeführt wird.

14. Verfahren nach einem der Ansprüche 1 bis 13, dadurch gekennzeichnet, daß die Mischung von Tetraalkoxytitan und Trialkylaluminium zusätzlich ein polares Modifiziermittel enthält, und das polare Modifiziermittel zum Trialkylaluminium zugegeben wird, gefolgt von der Zugabe des Tetraalkoxytitans, um die Mischung zu bilden.

15. Verfahren nach Anspruch 14, dadurch gekennzeichnet, daß das polare Modifiziermittel in der Katalysatormischung in einem Molverhältnis von ca. 1.3, bezogen auf das Tetraalkoxytitan, vorhanden ist.

16. Verfahren nach Anspruch 14 oder 15, dadurch gekennzeichnet, daß das polare Modifiziermittel in der Katalysatormischung in einem Molverhältnis von ca. 0.1, bezogen auf das Trialkylaluminium, vorhanden ist.

17. Verfahren nach einem der Ansprüche 14 bis 16, dadurch gekennzeichnet, daß das polare Modifiziermittel ausgewählt ist aus Alkyl- oder Arylphosphiten oder Phosphinen mit der allgemeinen Formel PR$_3$, worin R eine Phenoxy-, alkylsubstituierte Phenoxy-, Phenyl- oder Alkylgruppe bedeutet.

18. Verfahren nach einem der Ansprüche 14 bis 16, dadurch gekennzeichnet, daß das polare Modifiziermittel ausgewählt ist aus Stickstoffverbindungen der allgemeinen Formel NR$_3$, worin R ein Ethylradikal ist, aus aromatischen Stickstoff enthaltenden Verbindungen, wie z.B. Pyridinen, 4-alkylsubstituierten Pyridinen, 4-aminosubstituierten Pyridinen und 4-alkylaminosubstituierten Pyridinen, oder aus Wasser.

19. Verfahren nach einem der Ansprüche 1 bis 18, dadurch gekennzeichnet, daß zur Beendigung der Umsetzung Alkohol zur Reaktionsmischung zugegeben wird.

20. Verfahren nach Anspruch 19, dadurch gekennzeichnet, daß der Alkohol die allgemeine Formel ROH besitzt, worin R eine Alkylgruppe mit 1 bis 4 Kohlenstoffatomen ist.

**Revendications**

1. Procédé pour la dimérisation de l'éthylène en butène-1 qui comprend la mise en contact de l'éthylène à une température de 0 à 80°C sous une pression de 1 à 15 kg/cm$^2$ (0,98 à 14,7 bar) pendant 1 à 5 h avec un catalyseur qui est dépourvu d'ions chlorure et d'autres composés polaires et qui comprend un mélange de tétraalcoxytitane et de trialkylaluminium présents dans ledit mélange selon un rapport pondéral de l'aluminium au titane de 1 à 7,5, puis la séparation et la récupération du butène-1 ainsi produit.

2. Procédé selon la revendication 1, dans lequel ledit tétraalcoxytitane présente la formule générale Ti(OR)$_4$ dans laquelle R est un groupe alkyle, isoalkyle, aryle ou arylalkyle qui a de 1 à 10 atomes de carbone.

3. Procédé selon la revendication 2, dans lequel R dans la formule Ti(OR)$_4$ est un groupe butyle ou isopropyle.

4. Procédé selon l'une quelconque des revendications 1 à 3, dans lequel ledit trialkylaluminium présente la formule générale Al(R)$_3$ dans laquelle un ou plusieurs des R est un groupe alkyle, aryle ou alcoxy qui a de 1 à 6 atomes de carbone.

5. Procédé selon la revendication 4, dans lequel R dans la formule Al(R)$_3$ est un groupe éthyle ou isobutyle.

6. Procédé selon l'une quelconque des revendications 1 à 5, dans lequel le contact entre ledit catalyseur et ledit éthylène est réalisé en saturant ledit mélange catalytique par ledit éthylène.

7. Procédé selon l'une quelconque des revendications 1 à 6, dans lequel la réaction est réalisée en présence d'un solvant hydrocarboné.

8. Procédé selon la revendication 7, dans lequel ledit solvant est choisi parmi les hydrocarbures pa- raffiniques ayant de 5 à 14 atomes de carbone, les hydrocarbures chlorés ayant de 6 à 8 atomes de carbone et les hydrocarbures aromatiques ayant de 1 à 8 atomes de carbone.

9. Procédé selon la revendication 7 ou 8, dans lequel ledit solvant hydrocarboné est l'hexane ou l'heptane.

10. Procédé selon l'une quelconque des revendications 1 à 9, dans lequel ledit tétraalcoxytitane est mélangé avec ledit trialkylaluminium à une température d'environ 25 à 27°C.

11. Procédé selon l'une quelconque des revendications 1 à 10, dans lequel ladite réaction est réalisée à une température de 25 à 35°C pendant une durée d'environ 1 h.

12. Procédé selon l'une quelconque des revendications 1 à 11, dans lequel, lorsque le rapport pondéral de l'aluminium au titane est d'approximativement 4,3, ladite réaction est réalisée à une pression inférieure à 5 kg/cm$^2$ (4,9 bar).

13. Procédé selon l'une quelconque des revendications 1 à 11, dans lequel, lorsque ledit rapport pondéral de l'aluminium au titane est d'approximativement 6,4, ladite réaction est réalisée à une pression supérieure à 5 kg/cm$^2$ (4,9 bar).

14. Procédé selon l'une quelconque des revendications 1 à 13, dans lequel ledit mélange de tétraalcoxytitane et de trialkylaluminium comprend en outre un modificateur polaire, et ledit modificateur polaire est ajouté audit trialkylaluminium après quoi a lieu l'addition dudit tétraalcoxytitane pour former ledit mélange.

15. Procédé selon la revendication 14, dans lequel ledit modificateur polaire est présent dans ledit mélange catalytique en un rapport molaire d'approximativement 1,3 par rapport audit tétraalcoxytitane.

16. Procédé selon la revendication 14 ou 15, dans lequel ledit modificateur polaire est présent dans ledit mélange catalytique en un rapport molaire d'approximativement 0,1 par rapport audit trialkylaluminium.

17. Procédé selon l'une quelconque des revendications 14 à 16, dans lequel ledit modificateur polaire est choisi parmi les alkyl- ou arylphosphites ou phosphines présentant la formule générale PR$_3$, dans laquelle R est un groupe phénoxy, phénoxy alkyl-substitué, phényle ou alkyle.

18. Procédé selon l'une quelconque des revendications 14 à 16, dans lequel ledit modificateur polaire est choisi parmi les composés azotés de formule générale NR$_3$, dans laquelle R est un radical éthyle, les composés contenant de l'azote aromatique tels que les pyridines, les pyridines alkyl-substituées en position 4, les pyridines amino-substituées en position 4 et les pyridines alkylamino-substituées en position 4, ou l'eau.

19. Procédé selon l'une quelconque des revendications 1 à 18, dans lequel un alcool est ajouté audit mélange réactionnel afin d'arrêter la réaction.

20. Procédé selon la revendication 19, dans lequel ledit alcool présente la formule générale ROH, dans laquelle R est un groupe alkyle contenant de 1 à 4 atomes de carbone.